# FASCICULE DE BREVET EUROPEEN

(11) **EP 1 553 944 B1**
(45) Date de publication et mention de la délivrance du brevet: **27.02.2008**
(21) Numéro de dépôt: 03778429.5
(22) Date de dépôt: 21.10.2003
(51) Int. Cl.: A61K 31/444, A61K 31/4164, A61K 31/341, A61K 31/426, A61P 1/04

(54) **COMPOSITION PHARMACEUTIQUE ASSOCIANT LE TENATOPRAZOLE ET UN ANTAGONISTE DES RECEPTEURS H2 A L HISTAMINE**
PHARMAZEUTISCHE KOMBINATION VON TENATOPRAZOLE UND EINEM HISTAMIN-H2-REZEPTOR ANTAGONISTEN
PHARMACEUTICAL COMPOSITION COMBINING TENATOPRAZOLE AND A HISTAMINE H2-RECEPTOR ANTAGONIST

(30) Priorité: 21.10.2002 FR 0213114
(43) Date de publication de la demande: 20.07.2005
(73) Titulaire: Sidem Pharma, 2441 Luxembourg (LU); Mitsubishi Pharma Corporation, Osaka-shi, Osaka 541-0046 (JP)
(72) Inventeur: SCHUTZE, François, F-78860 SAINT-NOM-LA-BRETECHE (FR); CHARBIT, Suzy, F-94000 CRETEIL (FR); FICHEUX, Hervé, F-94130 NOGENT-SUR-MARNE (FR); HOMERIN, Michel, F-91080 COURCOURONNES (FR); TACCOEN, Alain, F-78150 LE CHESNAY (FR); INABA, Yoshio, Tokyo 103-8405 (JP)
(74) Mandataire: L'Helgoualch, Jean
(86) Numéro de dépôt international: PCT/FR2003/003124
(87) Numéro de publication internationale: WO 2004/037256

(56) Documents cités:
- UCHIYAMA ET AL.: "The long lasting effect of TU-199 a novel H+,K+-ATPase inhibitor on gastric acid secretion in dogs" J. PHARM. PHARMACOL., vol. 51, 1999, pages 457-464, XP008018962

## Description

La présente invention concerne une nouvelle association médicamenteuse, et plus particulièrement une nouvelle composition pharmaceutique combinant un antagoniste des récepteurs H2 à l'histamine et le ténatoprazole, pour le traitement des pathologies liées à l'hyperacidité gastrique, et plus particulièrement des ulcères gastriques et duodénaux, et des symptômes et lésions liés au reflux gastro-oesophagien.

Dans les traitements des troubles digestifs tels que la dyspepsie, l'hyperacidité gastrique, la gastrite, etc, on cherche généralement à éliminer l'acide gastrique qui est responsable de la dégradation de la muqueuse gastrique. Divers médicaments tels que des antiacides, des antagonistes des récepteurs H2 à l'histamine, et des inhibiteurs de la pompe à protons, ont été utilisés dans ces traitements.

Ainsi, les antagonistes des récepteurs H2 à l'histamine sont couramment utilisés pour le traitement des affections liées à une hypersécrétion d'acide gastrique, par exemple pour le traitement des ulcères gastriques car ils inhibent la sécrétion d'acide gastrique. L'antagoniste des récepteurs H2 à l'histamine peut être choisi parmi les produits bien connus comme la cimétidine, la ranitidine, la famotidine, etc.

Les inhibiteurs de la pompe à protons se sont aussi révélés utiles pour le traitement des ulcères gastriques. Le premier dérivé connu de cette série est l'oméprazole, décrit dans le brevet EP 005.129, qui possède des propriétés inhibitrices de la sécrétion acide gastrique, et est largement utilisé comme anti-ulcéreux en thérapeutique humaine. Parmi les autres inhibiteurs de la pompe à protons, on peut citer le rabéprazole, le pantoprazole, ainsi que le lansoprazole, qui présentent tous une analogie structurelle, et se rattachent au groupe des pyridinyl-méthyl-sulfinyl-benzimidazoles. Le ténatoprazole présente une structure analogue, mais du type imidazo-pyridine. Ces composés sont des sulfoxydes présentant une asymétrie au niveau de l'atome de soufre et sont donc généralement sous forme de mélange racémique de deux énantiomères.

L'oméprazole a aussi été envisagé dans le traitement des troubles du reflux gastro-oesophagien, mais son action dans une telle indication n'est pas totalement satisfaisante. Ainsi, des études ont montré que sa durée d'action, comme dans le cas des autres inhibiteurs de la pompe à protons, est insuffisante pour traiter efficacement le reflux nocturne.

Le ténatoprazole, c'est-à-dire la 5-méthoxy-2-[[(4-méthoxy-3,5-diméthyl-2-pyridyl)méthyl]sulfinyl]imidazo[4,5-b]pyridine, est décrit dans le brevet EP 254.588, ainsi que ses propriétés inhibitrices de l'ATPase (H⁺ + K⁺ et de la sécrétion d'acide gastrique (voir aussi Uchiyama et al. J. Pharm. Pharmacol., 1999, 51, 457-464).

Diverses associations de principes actifs appartenant à ces catégories ont aussi été envisagées pour tenter d'améliorer les effets pharmacologiques ou pour atténuer des effets secondaires connus. Par exemple, le brevet US 6.090.412 décrit une composition pharmaceutique pour administration orale associant un antagoniste des récepteurs H2 à l'histamine tel que la famotidine, avec au moins deux antiacides usuels tels que l'hydrogénocarbonate de sodium et l'hydroxyde de magnésium, qui ont un fort pouvoir de neutralisation, et un gel d'hydroxyde d'aluminium qui a un faible pouvoir de neutralisation. Le brevet FR 2.656.528 décrit l'association de la cimétidine et d'un agent antimuscarinique, la pirenzépine, qui est présentée comme diminuant les effets secondaires de la cimétidine.

Une étude a montré qu'il pourrait être utile d'administrer de l'oméprazole deux fois par jour et de la ranitidine le soir à des patients souffrant de reflux gastro-oesophagien (Peghini PL, Katz PO, Castell DO, "Ranitidine controls nocturnal gastric acid breakthrough on omeprazole: a control study in normal subjects" Gastroenterology (1998) 115(6):1335-9) mais d'autres études indiquent qu'un traitement comprenant l'administration d'oméprazole matin et soir était supérieur à un traitement complétant l'administration d'oméprazole par celle de ranitidine (Cross LB, Justice LN, "Combination of drug therapy for gastroesophagal reflux disease", Ann. Pharmacother. (May 2002) 36(5):912-6). Compte tenu de ces résultats, on peut donc penser que l'association d'un antagoniste des récepteurs H2 à l'histamine et d'un inhibiteur de la pompe à protons ne présente pas d'avantage particulier, sans doute en partie en raison de la faible demi-vie d'élimination de ces derniers.

Au contraire, les études réalisées par la demanderesse ont montré que l'association d'un inhibiteur spécifique de la pompe à protons, le ténatoprazole, et d'un antagoniste des récepteurs H2 à l'histamine procure des effets inattendus par rapport aux autres inhibiteurs de la pompe à protons et aux antagonistes des récepteurs H2 à l'histamine, utilisés isolément ou en association. Plus particulièrement, il a été montré que l'association du ténatoprazole et d'un ou plusieurs antagonistes des récepteurs H2 à l'histamine procure un contrôle de l'acidité gastrique bien supérieur à celui de chacun des composants utilisés isolément, et permet notamment de traiter efficacement des patients souffrant de symptômes de reflux gastro-oesophagien et réfractaires au traitement classique par un inhibiteur de la pompe à protons.

La présente invention a donc pour objet une composition pharmaceutique associant un inhibiteur de la pompe à protons spécifique, le ténatoprazole, et un ou plusieurs antagonistes des récepteurs H2 à l'histamine.

La présente invention a aussi pour objet une composition pharmaceutique pour administration par voie orale, comprenant du ténatoprazole et un ou plusieurs antagonistes des récepteurs H2 à l'histamine, sous une forme adaptée au traitement des pathologies liées à l'hyperacidité gastrique, notamment des ulcères gastriques et duodénaux, et des symptômes et lésions du reflux gastro-oesophagien.

L'invention a encore pour objet l'utilisation combinée du ténatoprazole et d'au moins un antagoniste des récepteurs H2 à l'histamine pour fabriquer un médicament pour le traitement des pathologies liées à l'hyperacidité gastrique, notamment des ulcères gastriques et duodénaux, et des symptômes du reflux gastro-oesophagien, ainsi que l'utilisation combinée du ténatoprazole et d'au moins un antagoniste des récepteurs H2 à l'histamine pour la fabrication d'un médicament destiné au traitement des pathologies liées à l'hyperacidité gastrique, notamment des ulcères gastriques et duodénaux, et des symptômes et lésions du reflux gastro-oesophagien.

Suivant l'invention, le ténatoprazole peut être utilisé sous forme libre ou sous forme de sel, et par exemple de sel de potassium, de magnésium, de sodium ou de calcium.

L'antagoniste des récepteurs H2 à l'histamine utilisé dans la composition de l'invention peut être choisi parmi la cimétidine, la ranitidine, la famotidine ou la nizatidine.

Le rapport en poids du ténatoprazole à l'antagoniste des récepteurs H2 à l'histamine peut être compris entre 1:30 et 1:2, et de préférence entre 1:20 et 1:5, ce rapport pouvant varier suivant l'antagoniste des récepteurs H2 à l'histamine choisi.

Des études antérieures ont montré que, à la fois chez des patients souffrant de symptômes de reflux gastro-oesophagien et chez des volontaires sains, environ 70% d'entre eux avaient un pic nocturne d'acidité, c'est-à-dire un pH inférieur à 4 pendant une durée d'au moins une heure au cours de la période nocturne entre 22 h et 6 h. On sait aussi que la gravité des lésions de la muqueuse de l'oesophage est liée à la durée de l'exposition au pH gastrique inférieur à 4.

Les nouvelles études effectuées ont montré que ces symptômes peuvent être traités efficacement avec une composition conforme à la présente invention, associant le ténatoprazole et un antagoniste des récepteurs H2 à l'histamine, et que cet avantage résulte d'une forme d'activité spécifique du ténatoprazole complétant celle de l'antagoniste des récepteurs H2 à l'histamine.

En effet, le ténatoprazole se distingue des autres inhibiteurs de la pompe à protons par une demi-vie d'élimination étonnamment plus longue, et aussi par une exposition tissulaire importante, comme l'ont démontré les expérimentations effectuées par la demanderesse.

Ainsi, l'étude de phase I chez des individus de type causasien (n=8 par groupe) a permis de montrer l'influence de différentes doses de ténatoprazole sur les paramètres pharmacocinétiques, dans le cas d'une administration par voie orale en une dose unique, et pendant une période de 7 jours.

Les doses testées sont de 10, 20, 40 et 80 mg de ténatoprazole.

Les résultats obtenus sont regroupés au Tableau 1 ci-après.

**Tableau 1**

| | Dose unique | | | | Dose répétée (7 jours) | | | |
|---|---|---|---|---|---|---|---|---|
| | 10 mg | 20 mg | 40 mg | 80 mg | 10 mg | 20 mg | 40 mg | 80 mg |
| Cmax (µg/ml) | 0,9 | 2,4 | 5,3 | 8,3 | 1,6 | 3 | 5,5 | 11,8 |
| Tmax (h) | 4 | 4 | 3 | 3 | 3 | 2 | 3 | 2 |
| T1/2 (h) | 5 | 6 | 6 | 7 | 5 | 8 | 9 | 9,2 |
| AUC 0-t | 8 | 24 | 43 | 97 | 13 | 36 | 75 | 218 |

Dans ce tableau, les abréviations utilisées ont les significations suivantes :
- Cmax: concentration maximale
- Tmax: temps pour obtenir la concentration maximale
- T1/2: temps de demi-vie d'élimination
- AUC₀₋ₜ: aire sous la courbe, entre le temps 0 et la dernière concentration mesurable.

Les résultats exposés au Tableau 1 ci-dessus montrent que les moyennes de temps de demi-vie d'élimination sont comprises entre 5 et 6 heures après administration d'une dose unique, et entre 5 et 9,2 heures après 7 jours d'administration, selon la dose. Le ténatoprazole présente aussi de fortes valeurs d'AUC (aire sous la courbe) mettant en évidence un faible taux de métabolisme et/ou une forte biodisponibilité par voie orale. De plus, quelles que soient les conditions d'administration, unique ou répétée, les valeurs de Cmax, AUC₀₋ₜ et AUC_{0-inf} augmentent de manière linéaire. La valeur de AUC_{0-inf} est calculée par extrapolation.

Une comparaison des valeurs d'AUC entre deux inhibiteurs de la pompe à protons, le lansoprazole et l'oméprazole, a déjà été faite par Tolman et al. (J. Clin. Gastroenterol., 24(2), 65-70, 1997) mais elle ne permet pas de juger de la supériorité d'un produit par rapport à un autre. En effet, différents critères entrent en jeu, à savoir le temps de régénération de la pompe, et le temps passé au-dessus de la concentration minimale nécessaire pour inhiber les pompes à protons. En ce qui concerne le temps de régénération des pompes, on observe que les pompes ont généralement une durée de demi-vie de l'ordre de 30 à 48 heures, et elles sont donc renouvelées totalement toutes les 72 à 96 heures.

L'étude pharmacocinétique réalisée par la demanderesse a montré que, grâce aux propriétés pharmacocinétiques inattendues exposées ci-dessus, le ténatoprazole permet de s'opposer au phénomène de régénération des pompes à protons en maintenant une concentration inhibitrice sur une période de temps suffisamment longue pour répondre aux deux critères précités.

Ainsi, l'exposition prolongée liée à la longue demi-vie du ténatoprazole, mise en évidence par la valeur d'AUC, lui confère une plus longue présence au niveau des sites d'action et procure donc un effet pharmacodynamique prolongé dans le temps. Les expérimentations montrent ainsi que le ténatoprazole possède un rapport demi-vie plasmatique / temps de régénération des pompes notablement plus élevé que celui des autres inhibiteurs de la pompe à protons, ce qui permet de l'utiliser dans des pathologies où les médicaments actuels sont peu efficaces, en particulier dans le traitement des symptômes nocturnes du reflux gastro-oesophagien, ainsi que des ulcères gastriques et duodénaux.

Aussi, lorsqu'il est associé à un antagoniste des récepteurs H2 à l'histamine, tel que la cimétidine et la ranitidine, de préférence par administration le soir au coucher, le ténatoprazole, par comparaison avec les autres inhibiteurs de la pompe à protons, procure un avantage significatif en ce qui concerne la suppression de l'acidité gastrique, et par conséquent permet une action efficace sur le pic nocturne d'acidité gastrique ainsi que sur les symptômes nocturnes chez les patients souffrant de reflux gastro-oesophagien, auquel il procure un soulagement important, même chez les patients réfractaires aux traitements classiques par des inhibiteurs de la pompe à protons usuels tels que l'oméprazole.

La composition de l'invention procure aussi un avantage sensible dans le traitement à la demande des symptômes du reflux gastro-oesophagien, indication où le volume de la prise de médicaments usuels doit être relativement important pour parvenir à une durée d'effet thérapeutique acceptable, contrairement à la présente invention.

La composition de la présente invention peut être administrée sous les formes usuelles adaptées au mode d'administration choisi, par exemple par voie orale ou parentérale, de préférence par voie orale ou intraveineuse. On peut utiliser par exemple des formulations de comprimés ou de gélules contenant le ténatoprazole et l'antagoniste des récepteurs H2 à l'histamine comme principes actifs, ou encore des émulsions ou solutions pour administration parentérale contenant un sel de ténatoprazole associé à un ou plusieurs antagonistes des récepteurs H2 à l'histamine, avec un support pharmaceutiquement acceptable usuel.

Les doses unitaires peuvent contenir entre 10 et 60 mg de ténatoprazole et entre 40 et 400 mg d'antagoniste des récepteurs H2 à l'histamine, en particulier la ranitidine ou la cimétidine.

A titre d'exemple, une formulation appropriée de gélule est indiquée ci-dessous :

| | | |
|---|---|---|
| Ténatoprazole | | 20 mg |
| Ranitidine | | 200 mg |
| excipients | q.s.p. | 300 mg |

La posologie est déterminée par le praticien en fonction de l'état du patient et de la gravité de l'affection. Elle est généralement comprise entre 10 et 120 mg, de préférence entre 20 et 40 mg, de ténatoprazole par jour, pour 200 à 400 mg de ranitidine.

Par exemple, un traitement des symptômes nocturnes du reflux gastro-oesophagien peut consister en l'administration de 1 à 2 comprimés contenant chacun 20 mg de ténatoprazole et 300 mg de ranitidine, chaque soir pendant une période de temps qui peut être comprise entre 4 et 10 semaines, dans le cas d'un traitement d'attaque ou d'entretien.

Dans le cas d'affections sévères, il peut être efficace d'administrer le médicament dans un premier temps par voie intraveineuse, puis par voie orale. L'invention présente en outre l'avantage de permettre un traitement séquentiel efficace par simple administration, par semaine, d'un seul comprimé dosé à 20 ou 40 mg de ténatoprazole associé à 20 à 300 mg d'antagoniste des récepteurs H2 à l'histamine par exemple la ranitidine ou la cimétidine.

L'étude de cas cliniques décrite ci-après a mis en évidence l'efficacité de la composition de l'invention.

**Tableau 2**

| Traitement de patients avec symptômes de reflux gastro-oesophagien | | | | |
|---|---|---|---|---|
| Age/Sexe | Symptôme prédominant | Durée de traitement | Evolution du symptôme | Tolérance |
| 47/M | b.n. | 8 semaines | ++ | +++ |
| 47/F | b.n. | 8 semaines | +++ | +++ |
| 39/F | b.n. | 4 semaines | ++ | +++ |
| 32/F | b.n. | 8 semaines | +++ | ++ |
| 45/M | b.n. | 8 semaines | +++ | +++ |
| 50/F | b.n. | 8 semaines | +++ | ++ |
| 34/M | b.n. | 4 semaines | +++ | +++ |
| 38/F | b.n. | 8 semaines | ++ | +++ |
| 46/M | b.n. | 8 semaines | +++ | +++ |

| | | | | |
|---|---|---|---|---|
| b.n.: brûlures nocturnes Les symboles +, ++ et +++ identifient une évolution du symptôme et une tolérance de valeur moyenne, favorable, et très favorable, respectivement. | | | | |

Le traitement consiste en une administration quotidienne, au coucher, d'un comprimé dosé à 20 mg de ténatoprazole et 300 mg de ranitidine. Le tableau 2 ci-dessus montre que le traitement est parfaitement toléré dans 7 cas sur 9 et bien toléré dans les deux autres, et que l'évolution constatée des symptômes a été généralement très favorable.

## Revendications

1. Composition pharmaceutique pour le traitement des pathologies liées à l'hyperacidité gastrique, **caractérisée en ce qu'**elle comporte en combinaison un ou plusieurs antagonistes des récepteurs H2 à l'histamine et le ténatoprazole.

2. Composition selon la revendication 1, **caractérisée en ce que** l'antagoniste des récepteurs H2 à l'histamine est choisi parmi la cimétidine, la ranitidine, la famotidine et la nizatidine.

3. Composition selon l'une quelconque des revendications 1 et 2, **caractérisée en ce que** le rapport en poids du ténatoprazole à l'antagoniste des récepteurs H2 à l'histamine est compris entre 1:30 et 1:2.

4. Composition selon l'une quelconque des revendications précédentes, **caractérisée en ce qu'**elle contient entre 10 et 60 mg de ténatoprazole et entre 40 et 400 mg d'antagoniste des récepteurs H2 à l'histamine.

5. Composition selon la revendication 1, **caractérisée en ce que** le ténatoprazole est sous forme de sel de potassium, de magnésium, de sodium ou de calcium.

6. Composition selon l'une quelconque des revendications précédentes, **caractérisée en ce qu'**elle est présentée sous une forme pour administration orale ou parentérale.

7. Utilisation combinée du ténatoprazole et d'au moins un antagoniste des récepteurs H2 à l'histamine pour la fabrication d'un médicament destiné au traitement des pathologies liées à l'hyperacidité gastrique.

8. Utilisation selon la revendication 7, **caractérisée en ce que** le médicament est destiné au traitement des ulcères gastriques et duodénaux, et des symptômes et lésions du reflux gastro-oesophagien.

## Claims

1. A pharmaceutical composition for the treatment of diseases related to gastric hyperacidity, **characterized in that** it contains a combination of one or more histamine H2-receptor antagonists and tenatoprazole.

2. The composition according to claim 1, **characterized in that** the histamine H2-receptor antagonist is selected from cimetidine, ranitidine, famotidine and nizatidine.

3. The composition according to either claim 1 or claim 2, **characterized in that** the weight ratio between tenatoprazole and the histamine H2-receptor antagonist is between 1:30 and 1:2.

4. The composition according to any of the preceding claims, **characterized in that** it contains between 10 and 60 mg of tenatoprazole and between 40 and 400 mg of a histamine H2-receptor antagonist.

5. The composition according to claim 1, **characterized in that** tenatoprazole is in the form of a potassium, magnesium, sodium or calcium salt.

6. The composition according to any of the preceding claims, **characterized in that** it is presented in a form for oral or parenteral administration.

7. The combined use of tenatoprazole and at least one histamine H2-receptor antagonist in the manufacture of a medicinal product intended for the treatment of diseases related to gastric hyperacidity.

8. Use according to claim 7, **characterized in that** the medicament is aimed at treating gastric and duodenal ulcers, and symptoms and lesions related to gastroesophageal reflux.

## Patentansprüche

1. Pharmazeutische Zusammensetzung zur Behandlung von Erkrankungen in Zusammenhang mit Magenübersäuerung, **dadurch gekennzeichnet, dass** sie eine Kombination aus einem oder mehreren Histamin-H2-Rezeptor-Antagonisten und Tenatoprazol umfasst.

2. Zusammensetzung nach Anspruch 1, **dadurch gekennzeichnet, dass** der Histamin-H2-Rezeptor-Antagonist aus Cimetidin, Ranitidin, Famotidin und Nizatidin ausgewählt ist.

3. Zusammensetzung nach einem der Ansprüche 1 und 2, **dadurch gekennzeichnet, dass** das Gewichtsverhältnis zwischen Tenatoprazol und dem Histamin-H2-Rezeptor-Antagonisten zwischen 1:30 und 1:2 liegt.

4. Zusammensetzung nach einem der vorangegangenen Ansprüche, **dadurch gekennzeichnet, dass** sie zwischen 10 und 60 mg Tenatoprazol und zwischen 40 und 400 mg des Histamin-H2-Rezeptor-Antagonisten enthält.

5. Zusammensetzung nach Anspruch 1, **dadurch gekennzeichnet, dass** das Tenatoprazol in Form des Kalium-, Magnesium-, Natrium oder Calciumsalzes vorliegt.

6. Zusammensetzung nach einem der vorangegangenen Ansprüche, **dadurch gekennzeichnet, dass** sie in einer Form zur oralen oder parenteralen Verabreichung vorliegt.

7. Verwendung einer Kombination aus Tenatoprazol und zumindest einem Histamin-H2-Rezeptor-Antagonisten zur Herstellung eines Medikaments zur Behandlung von Erkrankungen in Zusammenhang mit Magenübersäuerung.

8. Verwendung nach Anspruch 7, **dadurch gekennzeichnet, dass** das Medikament zur Behandlung von Magen- und Zwölffingerdarmgeschwüren und von durch gastroösophagealen Reflux hervorgerufen Symptomen und Läsionen dient.
